# EUROPEAN PATENT SPECIFICATION

(11) **EP 3 313 377 B1**
(45) Date of publication and mention of the grant of the patent: **21.08.2019**
(21) Application number: 16732720.4
(22) Date of filing: 23.06.2016
(51) Int. Cl.: A61K 9/127, A61K 45/06, A61K 31/047, A61K 31/407, A61K 31/427, A61K 31/496, A61K 31/546, A61K 31/685, A61K 31/7036, A61K 31/7048, A61K 35/12, A61P 31/04

(54) **COMPOSITIONS AND METHODS FOR USING LAMELLAR BODIES FOR THERAPEUTIC PURPOSES**
ZUSAMMENSETZUNGEN UND VERFAHREN ZUR VERWENDUNG VON LAMELLAREN KÖRPERN FÜR THERAPEUTISCHE ZWECKE
COMPOSITIONS ET PROCÉDÉS D'UTILISATION DE CORPS LAMELLAIRES POUR OBJECTIFS THÉRAPEUTIQUES

(30) Priority: 23.06.2015 GB 201511058
(43) Date of publication of application: 02.05.2018
(73) Proprietor: Lamellar Biomedical Limited, Eurocentral Holytown ML1 4WR (GB)
(72) Inventor: MCLEAN, Alec, Bellshill Central Scotland ML4 3NJ (GB); HOWARD, Lynsey, Bellshill Central Scotland ML4 3NJ (GB); KADIOGLU, Aras, Bellshill Central Scotland ML4 3NJ (GB); WINSTANLEY, Craig, Bellshill Central Scotland ML4 3NJ (GB)
(74) Representative: Murgitroyd & Company
(86) International application number: PCT/GB2016/051890
(87) International publication number: WO 2016/207645

(56) References cited:
- US-A- 5 925 375
- US-A1- 2005 070 877

## Description

### Field of the Invention

The present invention relates to the use of lamellar bodies in combination with an antibiotic to modulate quorum sensing to inhibit or reduce biofilm production. Biofilm production, which provides microbes with a multi-layered structured community attached to a surface and encased within a matrix of exopolymeric material, is associated with up-regulated virulence of infection and can limit the immune system response and/or the effectiveness of therapeutic agents. Thus, lamellar bodies have an important role to play in the treatment of microbial infections.

### Background to the Invention

It has been determined that blocking quorum sensing in bacteria can stop the cells from initiating behaviours, such as virulence factor production, that are synchronized through quorum sensing (Rutherford, S. T. and Bassler, B. L. Bacterial Quorum Sensing: Its Role in Virulence and Possibilities for Its Control Cold Spring Harbor Perspectives in Medicine. (2012)). Further, microbial infections where biofilms have formed become more clinically significant and can be difficult to treat with antibiotics. Persistent chronic infections often involve the development of microbial biofilms.

Biofilms are densely packed populations of microbial cells embedded within a self-produced extracellular polymeric matrix. This matrix of biopolymers such as DNA, proteins and polysaccharides, protects the microbial cells from dehydration, the immune system and antimicrobial agents. Biofilms can be associated with fungal species, such as *Candida albicans,* bacteria such as *Pseudomonas,* viruses such as HTLV-1 and protozoa such as *Acanthamoeba.*

The microbial cells growing in biofilms are physiologically distinct from planktonic cells of the same organism, which are single cells in a liquid medium. Clinically important bacteria such as *Pseudomonas, Burkholderia* and *Staphylococcus* spp. form biofilms that protect them from the host's immune defence and antibiotics. Biofilm formation has been associated with many chronic infections and diseases such as cystic fibrosis (CF) and infections associated with indwelling medical devices including stents, shunts, prosthetic joints, implants, endotracheal tubes and catheters. As well as shielding microbes from the immune system and anti-infective agents, biofilms also have the ability to clog pipes, watersheds, storage areas, and contaminate food products.

In general, bacteria have two life forms during growth and proliferation, cycling between periods when they perform individual behaviours and periods when they perform group behaviours. In the former, bacteria exist as single, independent, actively dividing and growing (planktonic) cells whereas, in the latter, they are organised into aggregations of cells with a reduced (sessile) metabolic activity, but an increased capacity to cause disease (virulence) through resilience to antibiotics or the immune defence system. These transitions are controlled by a cell-cell communication process called quorum sensing. Quorum sensing molecules are released, accumulate, and are synchronously detected by a group of bacteria resulting in community-wide changes in gene expression bacteria to collectively execute behaviours such as bioluminescence, biofilm formation and further virulence factor production.

There has been some evidence to suggest that decreased growth rates, as observed in sessile cells, can contribute to drug resistance. These sessile aggregates are commonly referred to as the biofilm growth phenotype, in recognition of the gene expression profile of the bacteria in this state. Biofilms form when bacteria adhere to surfaces in aqueous environments and begin to excrete a slimy, glue-like substance. Typically, the first bacterial colonists to adhere to a surface initially do so by inducing weak, reversible bonds called van der Waals forces. If the colonists are not immediately separated from the surface, they can anchor themselves more permanently using cell adhesion molecules. These bacterial colonists facilitate the arrival of other pathogens by providing more diverse adhesion sites. They also begin to build the matrix that holds the biofilm together. If there are species that are unable to attach to a surface on their own, they are often able to anchor themselves to the matrix or directly to earlier colonists.

The matrix or exopolymeric substance (EPS) of the biofilm that envelopes sessile communities of cells is considered to act as a barrier to the effective deployment of the host's natural defence mechanisms and as a barrier to diffusion of antibiotics and/or as a charged surface which binds antibiotics. Chronic biofilm-based infections are typically extremely resistant to antibiotics and many other conventional antimicrobial agents, additionally having an extreme capacity for evading the host defences. Biofilm antibiotic tolerance should not be confused with antibiotic resistance because, although bacteria within a biofilm tend to survive antibiotic treatment, they become susceptible to the treatment when the biofilm is disrupted or removed.

At present, to prevent or suppress bacterial biofilm infections, two methods are used: (i) early aggressive antibiotic treatment; and (ii) long term suppressive antibiotic treatment when the biofilm is established, if it cannot be removed physically. However, the administration of antibiotics to treat bacterial biofilms often demands combinations of several different antibiotics in high doses and for an extended period of time, because conventional resistance mechanisms will reinforce the intrinsic biofilm tolerance mechanisms. Therefore, there is a recognised need to develop a more effective method of treating microbial biofilm-associated infections which involves inhibiting the development of, disrupting or removing the bacterial biofilm so that the microbe can be destroyed by the host's immune system and/or antibiotics. The virulence profile of the infection, which is typically up-regulated in parallel with the development of biofilm and transition to a sessile state, is also diminished.

US5925375 discloses lamellar bodies for the treatment of inflammatory disorders which may be used in combination therapy; and a kit comprising the ingredients of the pharmaceutical composition.

### Summary of the Invention

The inventors have determined that lamellar bodies in combination with non-lamellar body antibiotics are capable of; interfering with the quorum sensing messaging system used by bacteria to change from planktonic to sessile phase; removing biofilms, in particular bacterial biofilms; inhibiting biofilm formation; and / or increasing bacterial cell wall permeability. In particular, the inventors have determined that administering lamellar bodies to a host subject suffering from microbial infection results in an improved immune response to said microbes and/or the potentiation of antibiotics provided to the host subject to inhibit the growth of, or destroy, the microbes.

Following extensive experimentation, the present inventors have identified that lamellar bodies in combination with non-lamellar body antibiotics can enhance the host's immune response by disrupting and/or removing biofilm, thus maintaining or reverting the microbe in/to a planktonic phase. Without wishing to be bound by theory, the inventors consider that the lamellar bodies disrupt biofilm, in particular bacterial biofilms and inhibit or interrupt bacterial production and/or release of quorum sensing molecules so that communication of the bacteria between each other, an essential precursor to the "decision" by bacteria to commence biofilm production and express virulence related genes, is inhibited. As a result, the biofilm is removed and its formation is reduced or prevented rendering bacteria more susceptible to the host's defences and/or anti-infective therapy.

According to a first aspect of the present invention there is provided a composition for use in the treatment of a microbial infection, as present in claim 1, comprising a therapeutically effective amount of lamellar bodies to inhibit or disrupt microbial quorum sensing. The composition is also useful in the disruption of existing or formation of microbial biofilm.

The composition is for use in the treatment of microbial infection in a host subject, in particular microbial infection associated with biofilm production in a host. In embodiments the lamellar body can act as a quorum sensing antagonist.

In embodiments, the lamellar bodies comprise phosphatidylcholine, sphingomyelin, phosphatidyl ethanolamine, phosphatidyl serine, phosphatidyl inositol and cholesterol. In particular, the lamellar bodies can comprise or consist in the range 44-70% phosphatidylcholine, in the range 15-23% sphingomyelin, in the range 6-10% phosphatidyl ethanolamine, in the range 2-6% phosphatidyl serine, in the range 2-4% phosphatidyl inositol and in the range 4-12% cholesterol by weight. In another preferred embodiment, the composition further comprises in the range 0-3% by weight of lysophosphatidyl choline.

In embodiments, the lamellar bodies comprise or consist of about 55% phosphatidylcholine, about 19% sphingomyelin, about 8% phosphatidyl ethanolamine, about 4% phosphatidyl serine, about 3% phosphatidyl inositol and about 10% cholesterol by weight (LMS-611 composition discussed herein). Specifically, in embodiments the lamellar body composition can comprise 55.1% PC, 19.4% SP, 8.2% PE, 4.1% PS, 3.1% PI and 10.1% CH. In an embodiment, the composition further comprises about 2% by weight of lysophosphatidyl choline.

In embodiments the biofilm can be present on an animal, suitably a mammal, in particular a human subject; a plant; or an inert material.

The composition of the present invention can effectively inhibit the formation of biofilms and reduce contamination by bacteria through application of the lamellar bodies to a device or site in which a biofilm is formed or forming.

In an embodiment of the present invention there is provided a composition for use in treating or preventing or slowing down a process or condition when the condition is caused by quorum sensing activity or signalling of microbes or bacteria.

In an embodiment, contact between the lamellar body and the object or subject to which the lamellar body is applied can be provided by spraying, dipping, brushing, or by other application of a solution including a lamellar body of the invention to a site to be treated.

According to the invention there is provided a composition comprising a therapeutically effective amount of lamellar bodies and at least a first non-lamellar body antimicrobial active agent, which is an antibiotic, for use in the treatment of a microbial infection.

In embodiments the lamellar bodies can be administered or provided as a composition for simultaneous, separate or sequential use with said antimicrobial agent.

According to a second aspect of the invention, there is provided a kit of parts comprising lamellar bodies and a combination treatment, which is an antibiotic for separate, sequential or simultaneous treatment of microbial infection of a host subject. Suitably, the kit comprises a therapeutically effective amount of antimicrobial agent. Suitably the antimicrobial agent or the lamellar bodies are formulated for use in relation to a specific site to be treated, for example for intranasal administration, for topical administration or for intravenous administration. Suitably, the antimicrobial agent or the lamellar bodies are formulated to be provided by different routes of administration to the subject to be treated.

In embodiments, the microbial biofilm is formed by a microbe selected from the group comprising bacteria, viruses, fungi, yeasts and protozoa. In embodiments the microbial biofilm can be formed by bacteria.

In embodiments the quorum sensing being disrupted can be from a Gram-positive bacterium. In embodiments the quorum sensing being disrupted can be from a Gram-negative bacterium.

In embodiments, the microbial infection can be an implant-associated and/or a catheter-associated and/or an indwelling medical device-associated infection.

It is well established in the literature that production of biofilm is a critical virulence factor for a number of commonly occurring life threatening infections including those caused by *Aspergillus fumigatus* and *Pneumocystis jirovecii.* Fungal biofilms are exopolysaccharide based, and it is considered they too will be affected by lamellar bodies, in particular the LMS-611 composition. Viruses, for example including HTLV-1, are also known to form biofilm and it is considered that lamellar bodies, in particular LMS-611 could be used to inhibit formation of such biofilms.

In embodiments, an antimicrobial active agent that is used in combination with a lamellar body can be any antibiotic or antibiotics, included but not limited to piperacillin, aztreonam, meropenem, gentamicin, tobramycin, erythromycin, tazocin, ceftazidime and ciprofloxacin or combinations thereof.

In embodiments the antibiotic can be selected from a group consisting of piperacillin, aztreonam, meropenem, gentamiycin, tobramycin, erythromycin, tazocin, ceftazidime and ciprofloxacin or combinations thereof.

Suitably lamellar bodies, in particular LMS-611, are provided to a bacterial colony prior to treatment with a combination therapy comprising an antibiotic, for example tobramycin, ceftazidime, tazocin or ciprofloxacin. Suitably, a concentration of at least 10 µg/ml. at least 15 µg/ml, at least 20 µg/ml, at least 30 µg/ml, at least 40 µg/ml of lamellar bodies are provided to the site to be treated. In embodiments, lamellar bodies, in particular LMS-611 can be provided prior to or simultaneously with, for example, tazocin, for example for treatment of infections of the lung.

Suitably the site to be treated may be the eye. Suitably the site to be treated may be the lung. Suitably, the lamellar bodies may be provided to the site to be treated by a different route of administration than the combination treatment, for example the antibiotic(s) being provided. For example the lamellar bodies may be provided intranasally whilst the antibiotic is provided intravenously.

Quorum sensing and in some cases biofilm production can be problematic where microorganisms are grown in sufficient density to allow for high yield production of fermentation products. An ability to modulate quorum sensing to allow an increase in cell density in such production facilities and to minimise biofilm production may be advantageous.

Accordingly, there is provided, without being claimed, a method of increasing the volumetric productivity of a population of known microorganisms, the method comprising:
a) introducing lamellar bodies to a culture medium of microorganisms, for example bacteria, capable of forming biofilm, and
b) growing the microorganisms, for example bacteria;
wherein the volumetric productivity of the microorganisms is greater in the presence of the lamellar bodies with respect to a fermentation product produced by the microorganisms than the volumetric productivity of the fermentation product of the microorganism in the absence of lamellar bodies.

There is also provided, without being claimed, a method of increasing the cell density of a population of known microorganisms, said method comprising:
a) introducing lamellar bodies to a culture medium of microorganisms, for example bacteria, capable of forming biofilm
b) growing the microorganisms in the culture medium including lamellar bodies, wherein the microorganisms when grown in a culture including lamellar bodies grow to a greater cell density than the cell density of a culture medium of otherwise identical microorganisms that does not include the lamellar bodies and is cultured under identical culture conditions.

There is also provided, without being claimed, a method of producing a fermentation product, said method comprising:
a) providing a genetically modified known microorganism, for example bacteria, capable of producing a fermentation product,
culturing the modified microorganism in a culture medium wherein when the culture medium is provided with lamellar bodies the genetically modified microorganism has the ability to achieve a higher volumetric productivity for a fermentation product than the volumetric productivity for the same fermentation product when produced in a culture medium which does not include the lamellar bodies.

In illustrative embodiments the method can include the further step of harvesting at least one fermentation product from the culture medium.

### Brief Description of the Figures

Embodiments of the present invention will now be described by way of example only with reference to the figures described below.

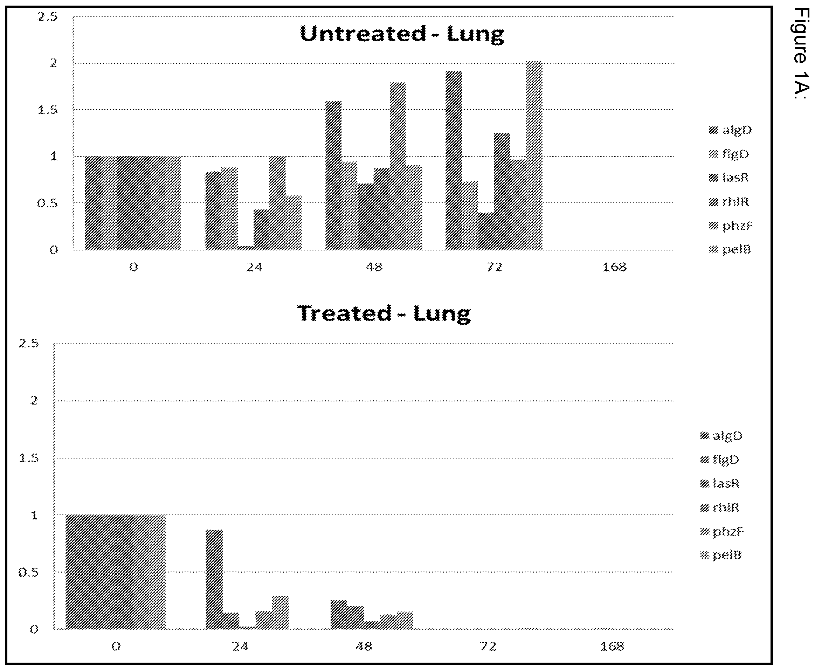

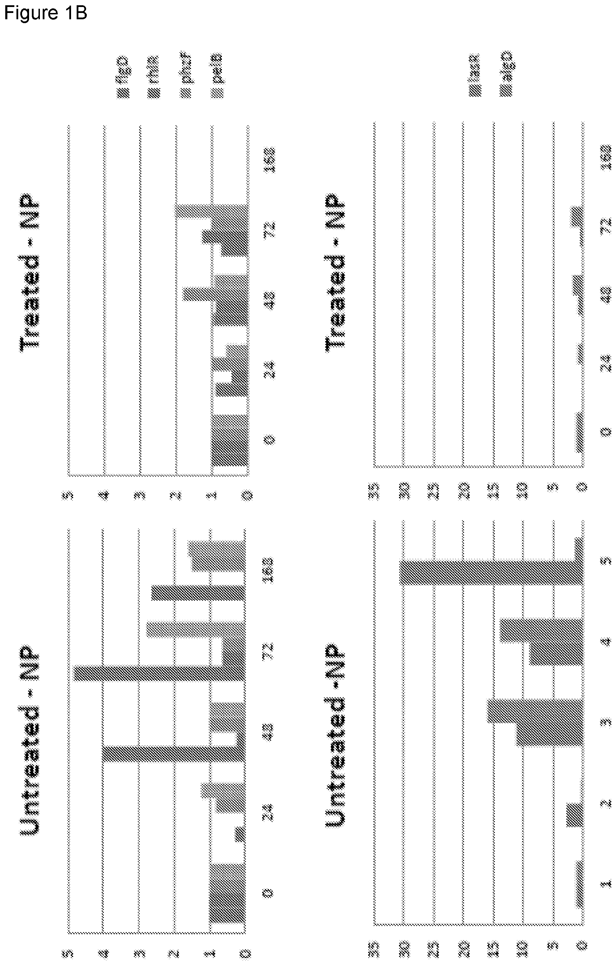

Figure 1 illustrates (A) gene expression data after LMS-611 treatment wherein mice lungs were infected intranasally with a fresh mid-log phase dose of 2 x 10⁶ CFU *P. aeruginosa* strain LESB65 in 50 µl phosphate-buffered saline (PBS). RNA isolated from mouse tissue at hour 0, 24, 48 and 72, and 168 was treated to remove all host RNA and bacterial RNA tested for virulence factors algD, flgD, lasR, rhlR, phzF and pelB, and (B) illustrates gene expression of P. *aeruginosa* in the nasopharynx wherein in the untreated samples flgD and pelB shows a marked increase in expression during the seven day period (point 1 time 0, point 2 time 24h, point 3 time 48 h, point 4 time 72 h and point 5 time 168 h (7 days)) and there is also an increase in pelB in the treated samples, but this is reduced compared to the control. The untreated samples show very large increases in algD and lasR, not seen in the LMS-611 treated samples;

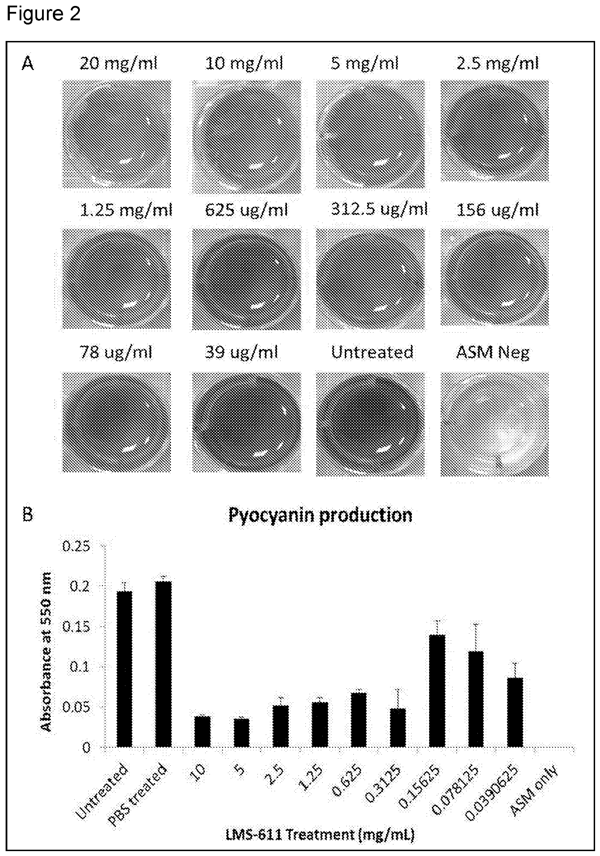

Figure 2 illustrates pyocyanin levels detected in artificial sputum cultures after LMS-611 treatment wherein bacteria were grown into a biofilm state for 3 days and then treated for 24 hours with varying concentrations of LMS-611 with images taken of the ASM biofilm assay (A) with quantification of pyocyanin production by absorbance at 550 nm (B). n = 2;

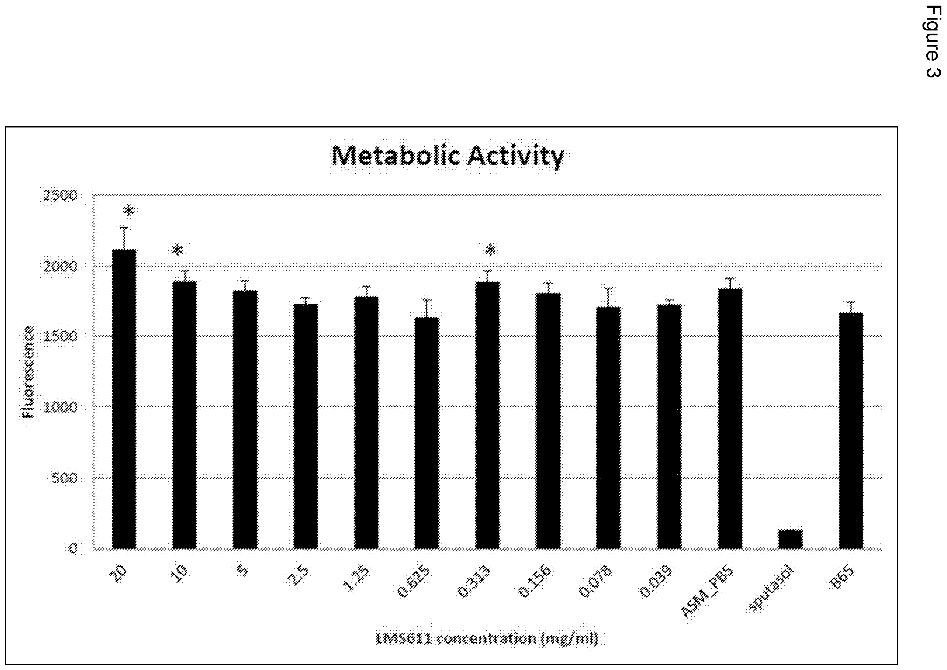

Figure 3 illustrates *P. aeruginosa* (PA) remains in an active growth phase where they are metabolically active after being treated with LMS-611 wherein Resazurin is a dye that measures the metabolic activity of microbes. Biofilms were grown for 3 days, then differing concentrations of LMS-611 were added for a further 24 hours. ASM is artificial sputum medium. Cultures were then homogenised using Sputasol and Resazurin added and measurements were taken after incubation at 37 °C for 1 hour (Non-parametric statistical tests were performed (ANOVA on ranks and Dunn's post hoc test) and each treatment was compared to the B65 control. * = p<0.05);

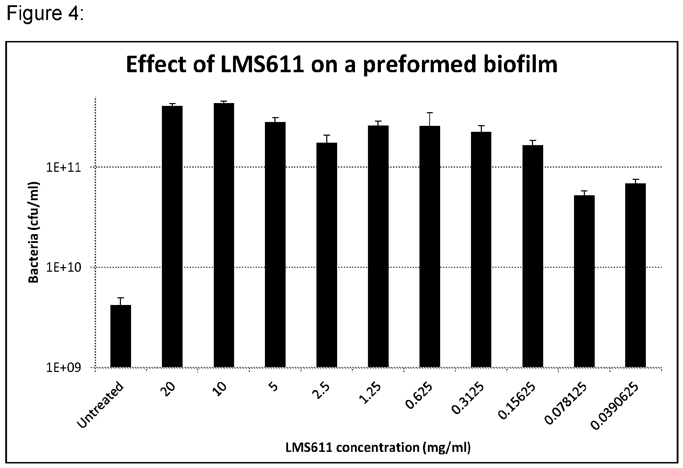

Figure 4 illustrates the effect of LMS-611 on PA in biofilms grown for 3 days and then treated for 24 hours with varying concentrations of LMS-611. Images of PA biofilm in ASM. Quantification of bacterial count from the ASM biofilm (CFU/mL). An ANOVA on ranks and Dunn's post hoc test were used to determine whether the cfu/ml of each treatment was significantly different to the control.* = p<0.05;

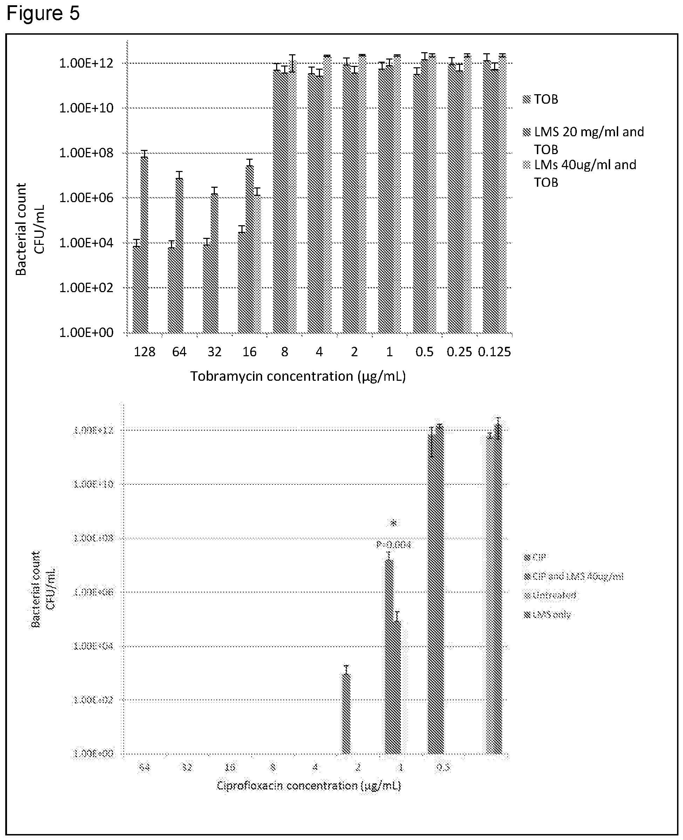

Figure 5 illustrates antibiotic potentiation wherein a range of concentrations of bacteria were grown into a biofilm for 3 days and then treated for 24 hours with varying concentrations of LMS-611 and/or antibiotics tobramycin (A) and ciprofloxacin (B). An ANOVA on ranks and Dunn's post hoc test were used to determine whether the cfu/ml of each treatment was significantly different to the control.* = p<0.05;

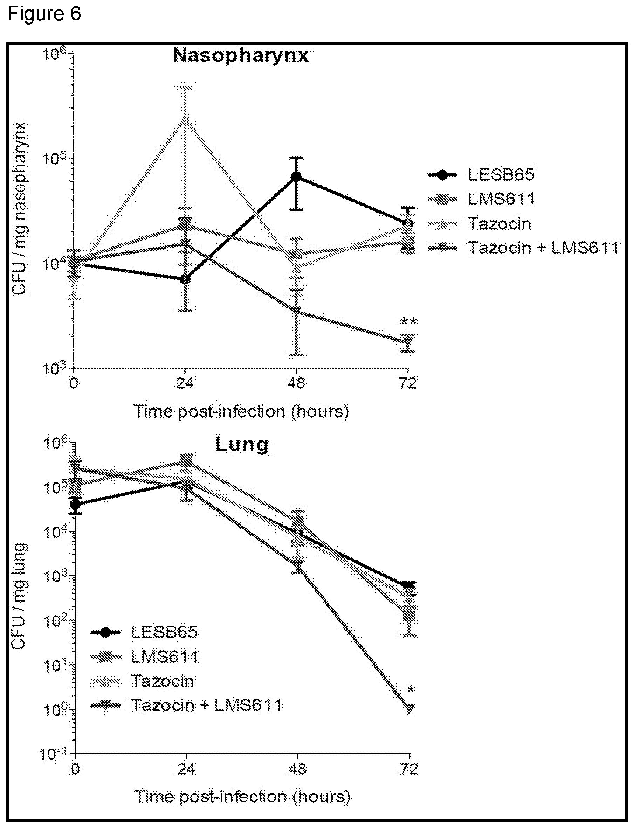

Figure 6 illustrates quantification of PA in a mouse infection model wherein mice lungs were intranasally infected with LESB65 (2X106 CFU in 50ul PBS) and given intranasal LMS-611 (1mg per dose) and/or intravenous Tazocin (2mg piperacillin and 0.5mg tazobactam per dose) at 24 and 48 hours post-infection. Bacteria were quantified from nasopharynx (A) and lung tissue (B). Data are from 5 mice per group. * = p<0.05 and ** =p<0.01 in two-way ANOVA analysis;

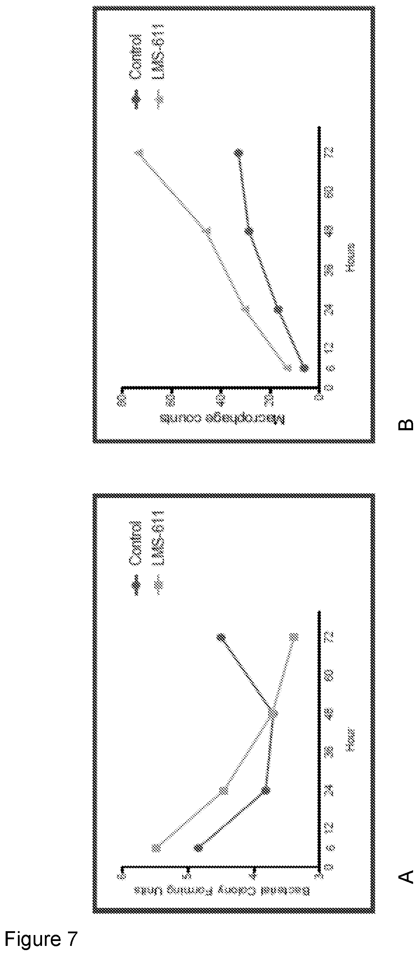

Figure 7 illustrates (A) that in-vivo testing of LMS-611 can significantly reduce *Pseudomonas* Colony Forming Units (CFUs) in a murine lung model (p=0.01 at day 3) and (B) that there is a significant increase in macrophage response to *Pseudomonas* infection with LMS-611 (p=0.05 for days 1 to 3, inclusive);

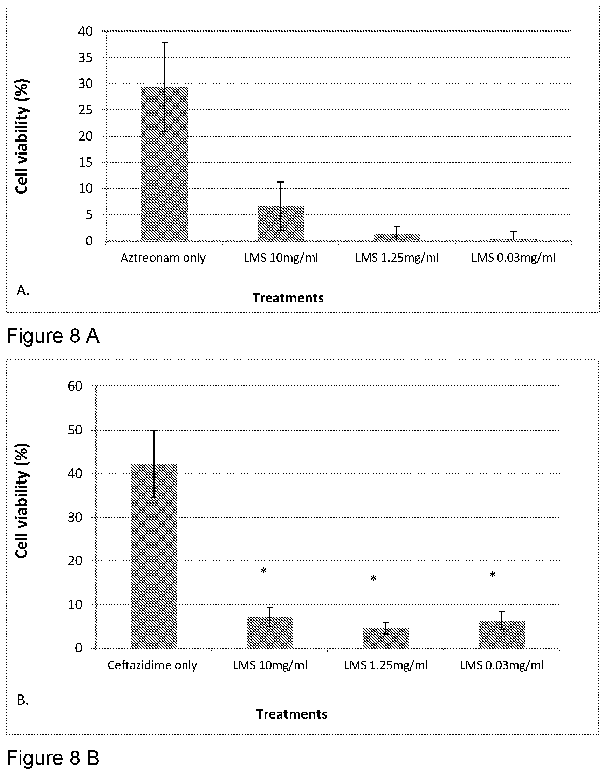

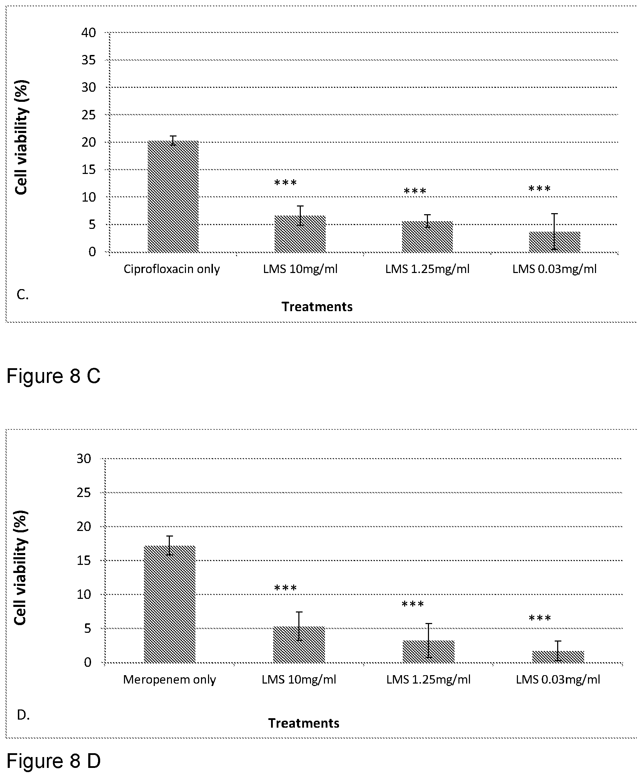

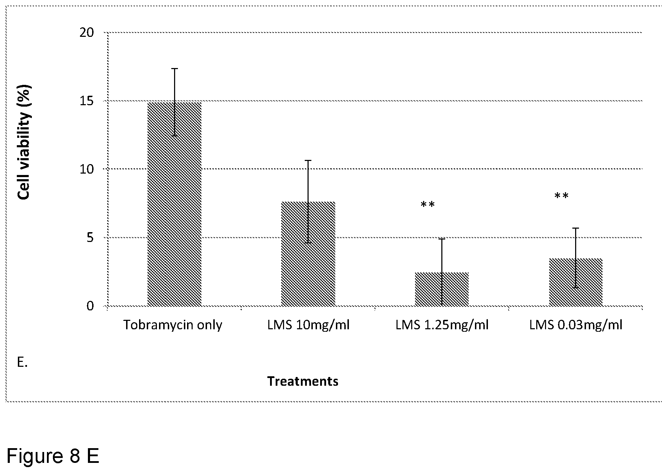

Figure 8. *P*. *aeruginosa* (strain PA01) biofilms were formed for 24h on polystyrene pegs. Biofilms were treated for 1h with LMS-611 at three different concentrations (10mg/ml, 1.25mg/ml and 0.03mg/ml). Biofilms were then treated with the clinically important antibiotics aztreonam (A), ceftazidime (B), ciprofloxacin (C), meropenem (D) and tobramycin (E) at a concentration of 1 x MIC (minimum inhibitory concentration) for 18h. The viability of biofilm-associated cells was assessed following treatment using a metabolic XTT assay and treated biofilms were compared to untreated control biofilms to calculate the cell viability (%). Error bars represent the standard deviation between replicate biofilms. Results were analysed by performing an unpaired, two-tailed T-test comparison between control and treated biofilms. *=P≤0.05, **=P≤0.001, ***=P≤0.0001.

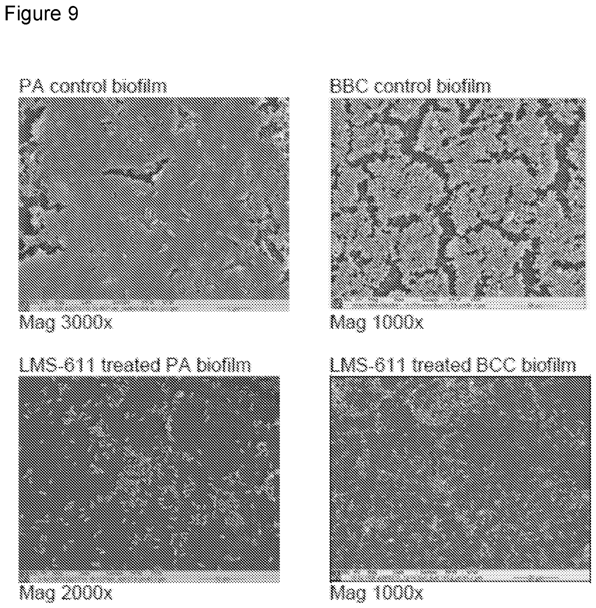

Figure 9 illustrates a composition of scanning electron micrographs for both PA and *Burkholderia cepacia* complex (BCC) control strains and LMS-611 treated PA and BCC strains revealing in LMS-611 treated strains, biofilms were scant and disrupted.

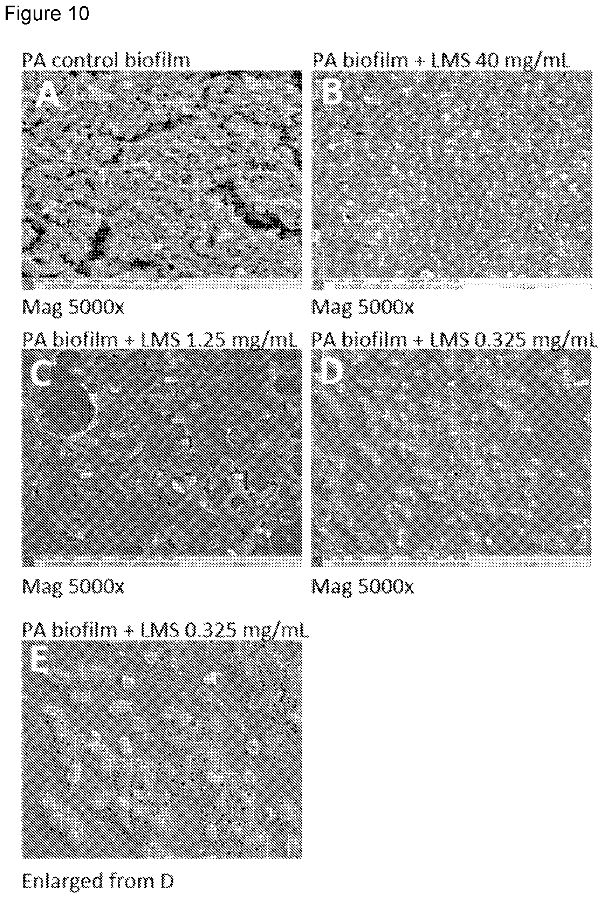

Figure 10 Scanning electron microscopy (SEM) images (5000 x magnification) of untreated biofilms of PA01 (A) grown for 24h and biofilms of PA01 grown for 24h treated with LMS-611 reconstituted in physiological saline at 40mg/ml (B), 1.25mg/ml (C) and 0.325mg/ml (D & E) for 18h. Biofilms were fixed and coated with gold then imaged on a JEOL 6400 scanning electron microscope. The untreated control biofilm (A) shows multiple layers of rod-shaped *P. aeruginosa* cells encased within the web-like matrix of the biofilm which consists of polysaccharides, proteins and DNA. In the biofilm treated with the highest dose of LMS-611 (40mg/ml) (B), the LMS appears to coat the surface of the biofilm and there seems to be a reduction in the 3D structure. The biofilms treated with 1.25mg/ml LMS-611 (C) and 0.325mg/ml (D) displayed a clear reduction in the number of cells present in comparison to the multi-layered untreated biofilm (A). In the magnified image of the biofilm treated with 0.325mg/ml (E), not only are the number of *P. aeruginosa* cells reduced but the cells that remain have an altered morphology. Instead of healthy rod-shaped cells many of them have become damaged by treatment.

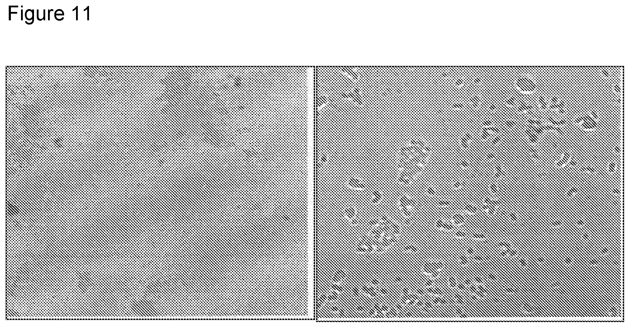

Figure 11 illustrates that incubation of *Pseudomonas* cells (magnification on left hand side x 100 and right hand side x 1200) with LMS-611 rendered bacterial membranes permeable to propidium iodide, as evidenced by their fluorescent staining (left image is propidium iodide alone and right image is propidium iodide and LMS-611);

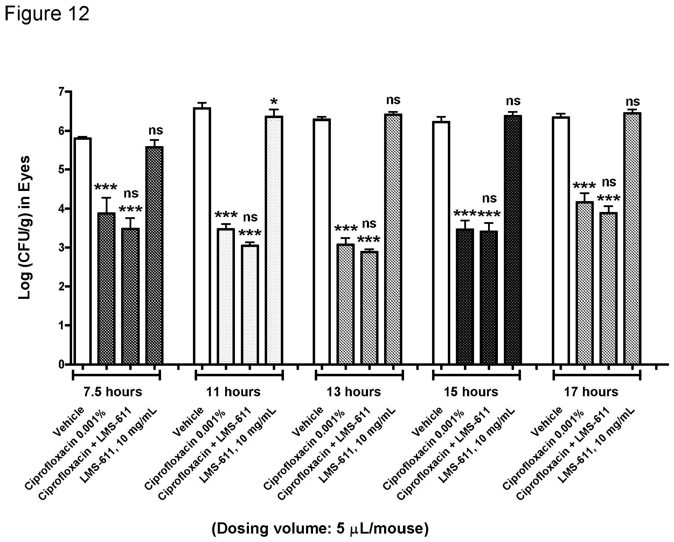

Figure 12 illustrates the result of the effects of ciprofloxacin and LMS-611 against *P. aeruginosa* ATCC 15692 (PA01) eye infection in female C57BL/6 mice. On Day 0, animals were inoculated ocularly with *Pseudomonas aeruginosa* suspensions in 5µL PBS. Test substances were administered to groups of 5 animals 5 and 10 h after bacterial inoculation. The individual eyes were surgically harvested from euthanized animals 7.5, 11, 13, 15 and 17 h after inoculation. The eyes were homogenized in 1mL PBS and dilutions were plated on MacConkey agar plates for CFU determination. CFU/g eye was calculated for each collecting time point. Data were displayed as Mean +/-SEM. One-way ANOVA and Tukey's multiple comparison test were applied for comparison between the treated and vehicle groups at each measurement time point, and applied to the comparison between the ciprofloxacin only and ciprofloxacin combined with LMS-611 groups. *: p<0.05 Treated vs. Vehicle, **: p<0.001 Treated vs. Vehicle, ***: p<0.0005 Treated vs. Vehicle, ns: no significant effect - Dosing volume was 5µL per mouse;

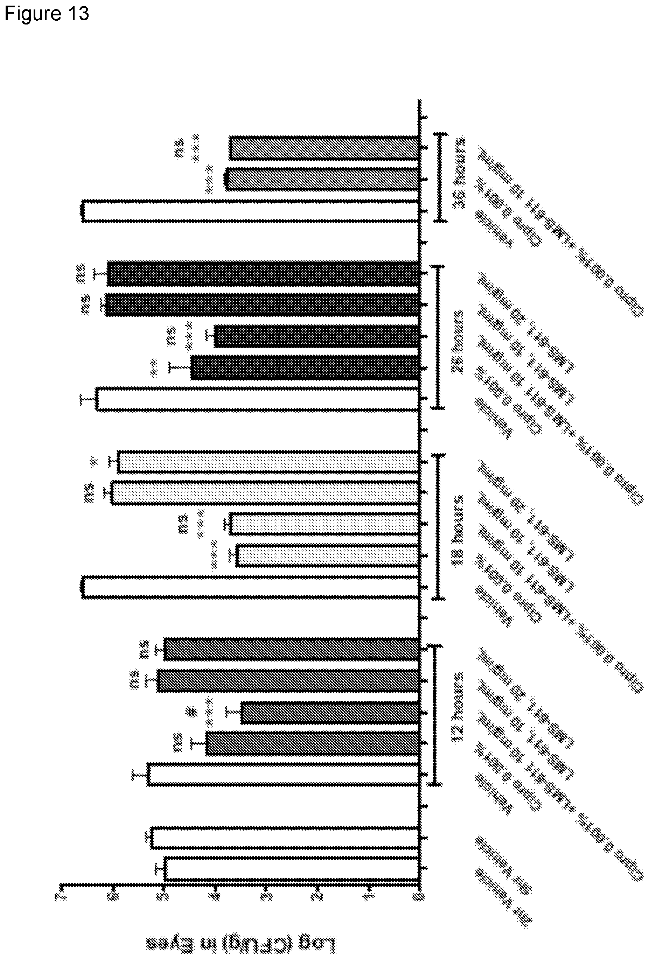

Figure 13 illustrates the effects of ciprofloxacin and LMS-611 against P. *aeruginosa* ATCC 15692 (PA01) eye infection in female C57BL/6 mice. On day 0, animals were inoculated ocularly with suspensions of *P. aeruginosa* cells in 5µL PBS. Test substances were administered 5 and 10 h after bacterial inoculation, five animals per group. The individual eyes were surgically harvested from euthanized animals 12, 18 or 26 h after inoculation. The eyes were homogenized in 1mL PBS and dilutions were plated on MacConkey agar plates for CFU determination. CFU/g eye was calculated for each collecting time point. Data were displayed as +/- SEM. One-way ANOVA and Tukey's multiple comparison test were applied for comparison between the treated and vehicle groups at each measurement time point, and applied to the comparison between the ciprofloxacin only and ciprofloxacin combined with LMS-611 groups. *: p<0.05 vs Vehicle, **: p<0.001 vs Vehicle, ***: p<0.0005 vs Vehicle, #: p<0.05 vs ciprofloxacin, ns: no significant effect. Cipro: ciprofloxacin;

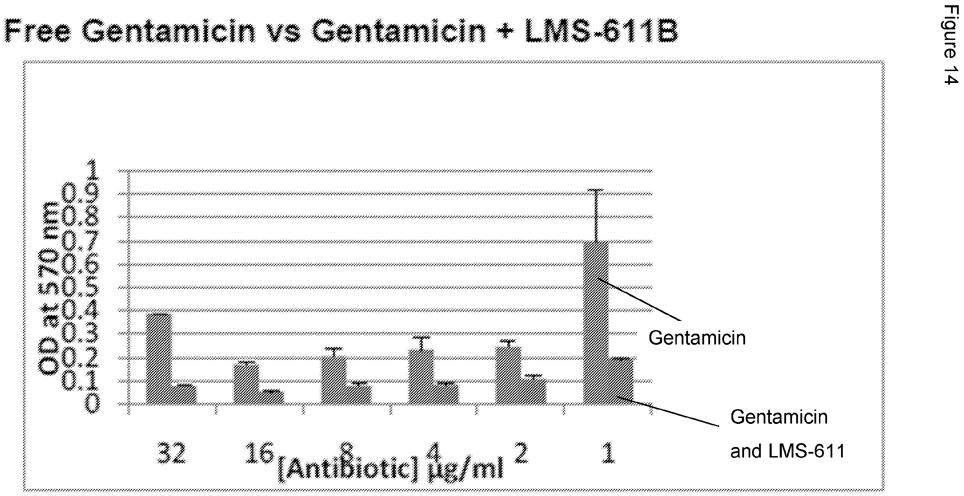

Figure 14 illustrates the effect of LMS-611 with gentamicin in reducing biofilm MICs of *Pseudomonas aeruginosa* strain PA01 and that this is more effective that gentamicin alone;

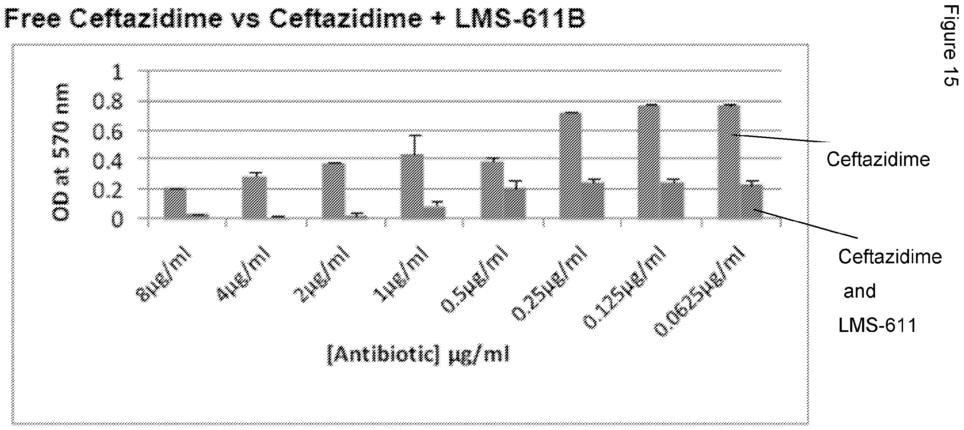

Figure 15 illustrates the effect of LMS-611 with ceftazidime in reducing biofilm MICs of *Pseudomonas aeruginosa* strain PA01 and that this is more effective that ceftazidime alone;

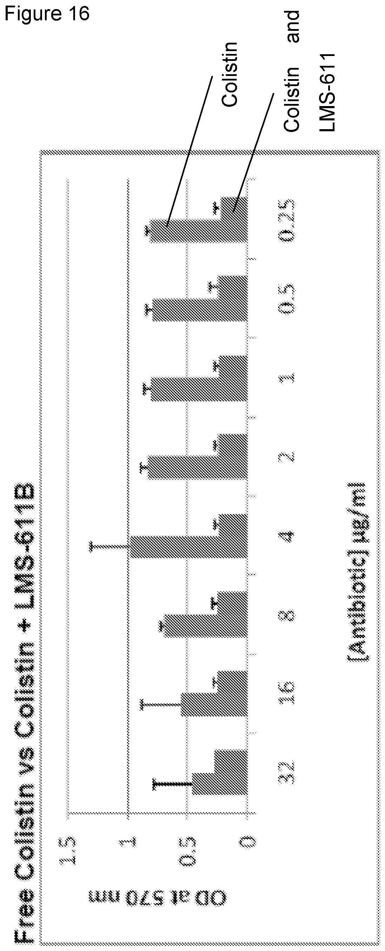

Figure 16 illustrates the effect of LMS-611 with colistin in reducing biofilm MICs of *Pseudomonas aeruginosa* strain PA01 and that this is more effective that colistin alone;

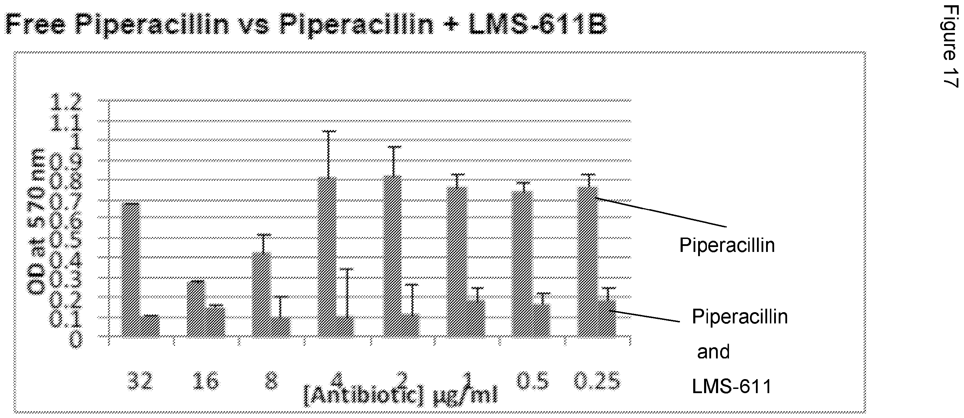

Figure 17 illustrates the effect of LMS-611 with piperacillin in reducing biofilm MICs of *Pseudomonas aeruginosa* strain PA01 and that this is more effective that piperacillin alone

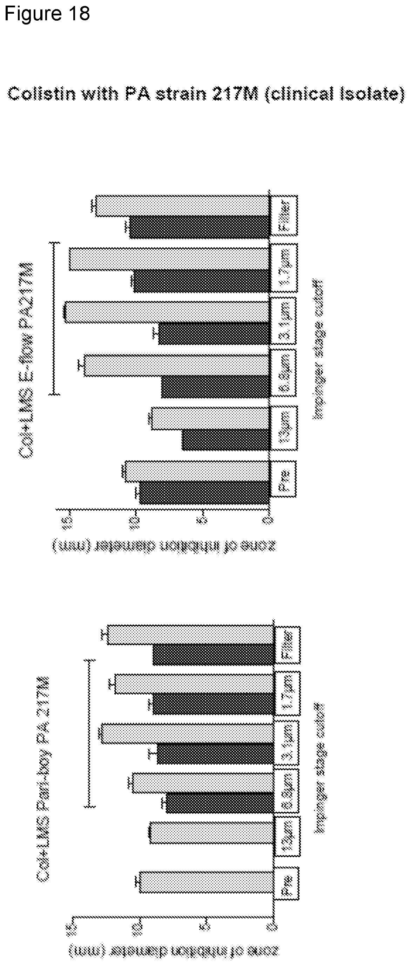

Figure 18 illustrates the effect of nebulisation of LMS-611 when combined with Colistin with PA strain 217M using nebulisers with two different modes of operation (Pari-boy (jet-type) and Pari Flow (oscillating mesh)) wherein a multistage liquid impactor (MSLI) was used to measure the aerodynamic particle size distribution and deposition from nebulised LMS-611 compositions wherein the MSLI from Copley measures particle size in accordance with the European Pharmacopeia guidelines (Chapter 29.9.18) and the impactor has five stages simulating the various parts of the human respiratory system. Particles with the same inertia will impact upon a particular stage, whilst smaller particles will pass onto the next impaction stage such that by analysing the amount of LMS-611 deposited on the various stages the fine particle dose and fraction can be calculated wherein the fine particle dose (respirable fraction) is the proportion of the dose from an inhalation device (nebuliser) that is made available to the airways, and this correlates with the dose of LMS-611 that reaches the conducting airways during inhalation;

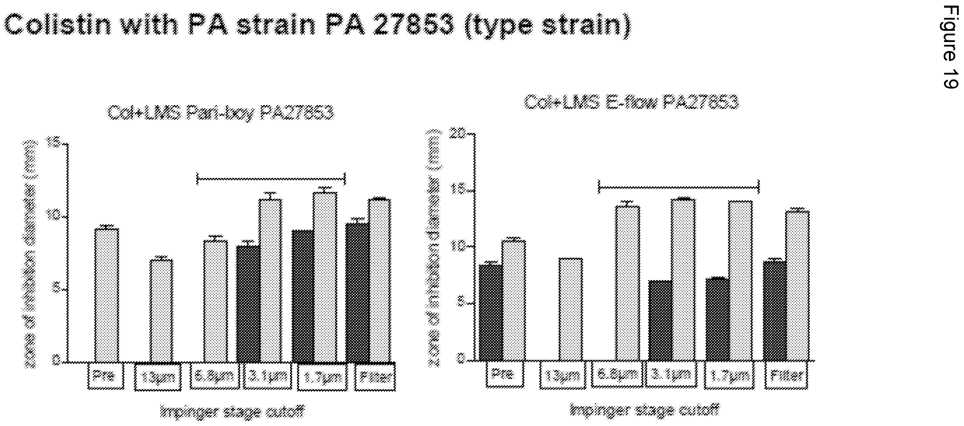

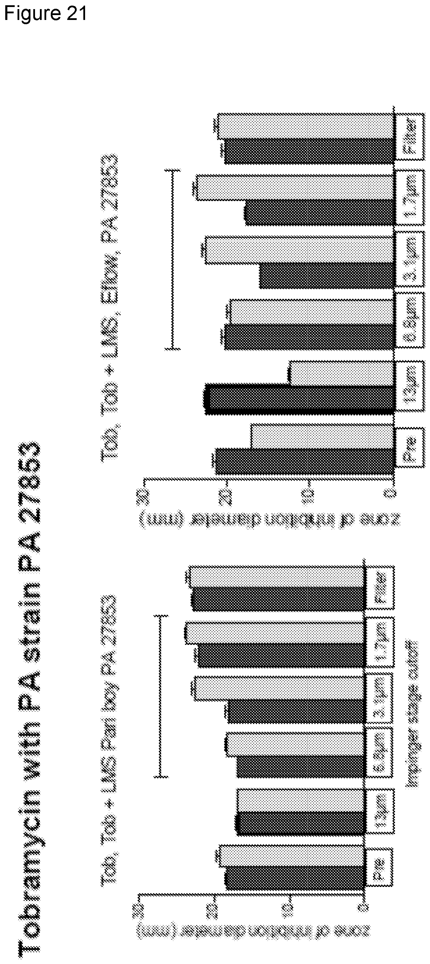

Figure 19 illustrates the effect of nebulisation of LMS-611 when combined with colistin in relation to PA2783 strain and as per Figure 21 illustrates colistin is delivered more efficiently when combined with LMS-611 and that the combination has its greatest bactericidal effect of the MLSI stages which represent divisions of the lung which are the site of cystic fibrosis lung disease;

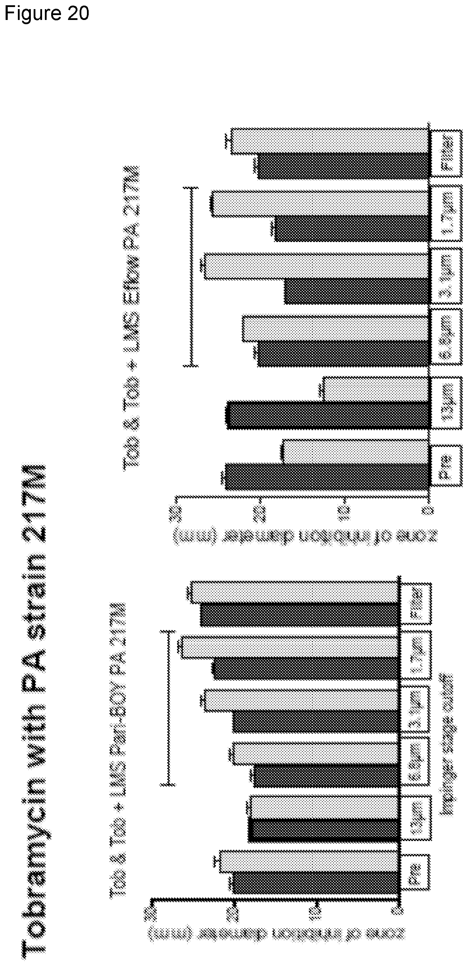

Figure 20 illustrates the effect of nebulisation of LMS-611 when combined with tobramycin with PA strain 217M
Figure 21 illustrates the effect of nebulisation of LMS-611 when combined with tobramycin with PA strain 27853 and demonstrates that tobramycin in combination with LMS-611 is delivered to all stages of MLSI and the bactericidal effects of the combination appear to be greater on the 6.8 µm, 3.1 µm and 1.7 µm stages of the impactor which would represent the site of cystic fibrosis lung disease.

### Detailed Description of the Invention

The inventors of the present invention have surprisingly discovered that administration of lamellar bodies in combination with an antibiotic to bacteria results in a gene expression profile consistent with a planktonic rather than sessile state. Without being bound by theory, the inventors consider that the lamellar bodies disrupt the formation of biofilm and down-regulate virulence associated gene expression, thus diminishing the pathogenicity of the bacteria and enabling the host subject's immune response and/or antimicrobial agents to access microbes. Thus, the action of the lamellar bodies to inhibit formation of biofilm is considered to allow bacteria to be inhibited and/or killed by the immune system and/or anti-infectives such as antibiotics. The inventors have also demonstrated that the availability of pyocyanin produced by *Pseudomonas aeruginosa* is diminished in vitro under the effect of lamellar bodies; pyocyanin being a key virulence factor produced by the bacteria under the control of quorum sensing mechanisms. The inventors have demonstrated that administration of lamellar bodies to a site of microbial infection can lead to a significant increase in the number of macrophages recruited to the site of infection. It is further postulated that lamellar bodies can alter the permeability of bacterial walls rendering them more susceptible to the host's immune response and to antibiotics.

Although not wishing to be bound by theory, it is considered that the microbes, in particular bacteria, that become part of a biofilm engage in quorum sensing - a type of cell to cell communication that supports microbial cellular processes. Although the mechanisms behind quorum sensing are not fully understood, it is considered the communication process allows, for example, a single-celled bacterium to perceive the proximity of other bacteria. If a bacterium can sense that it is surrounded by a sufficiently dense population of other pathogens, it becomes stimulated to contribute to the formation of a biofilm and the production of pyocyanin and other virulence factors through modifications in gene expression. It is considered that lamellar bodies may have the ability to sequester and quench the signal of quorum sensing molecules such as N-acyl homoserine lactones (AHL).

The inventors have demonstrated that administrating lamellar bodies in combination with an antibiotic to a host, and in particular LMS-611, results in the improved clearance of microbial infection by cells of the immune system of the host. In embodiments, a lamellar body comprises a phospholipid composition in the range 44-77% phosphatidyl choline (PC), in the range 15-23% sphingomyelin (SP), in the range 6-10% phosphatidyl serine (PS), in the range 2-4% phosphatidyl inositol (PI) and in the range 4-12% cholesterol (CH) by weight, in particular LMS-611 lamellar bodies comprise about 55% PC, 19% SP, 8% PE, 4% PS, 3% PI and 10% CH.

LMS-611 appears to potentiate a range of antibiotics by reducing the minimum inhibitory concentrations (MIC - the lowest concentration of an antimicrobial inhibiting visible growth of a microorganism) of antibiotics to bacteria giving scope to improve bacterial clearance and/or use of LMS-611/antibiotic combinations at lower doses (or via different routes) with the same efficacy. Given that the use and dose of antibiotics are often restricted by their side effect and/or toxicity profile, this also increases the potential use of potent antibiotics the use of which would be otherwise limited.

### Definitions

As used in this specification and the appended claims, the singular forms "a", "an", and "the" include plural references unless the context clearly dictates otherwise. Thus, for example, references to "the method" include one or more methods, and/or steps of the type described herein and/or which will become apparent to those persons skilled in the art upon reading this disclosure and so forth.

Unless defined otherwise, all technical and scientific terms used herein have the same meaning as commonly understood by one of ordinary skill in the art to which this invention belongs. Although any methods and materials similar or equivalent to those described herein can be used in the practice or testing of the invention, the preferred methods and materials are described. "Lamellar bodies" or "microbodies" as used throughout this document, refers to phospholipid, multilamellar, bilayered structures.

"Treat", Treating" or "Treatment" refers to therapy, prevention and prophylaxis and particularly refers to the administration of medicine or the performance of medical procedures with respect to a subject, for either prophylaxis (prevention) or to cure or reduce the extent of or likelihood of occurrence of the infirmity or malady or condition or event in the instance where the subject is afflicted. A "therapeutically effective amount" is an amount sufficient to decrease or prevent the symptoms associated with the conditions or deficiencies contemplated for therapy with the compositions of the present invention. "Combination therapy" refers to the use of the agents of the present invention with other active agents or treatment modalities. These other agents or treatments may include drugs such as antimicrobials, in particular antibacterial agents, for example antibiotics, antifungal agent, antiviral agents or anti protozoal agents; corticosteroids, non-steroidal anti-inflammatory compounds, or other agents useful in treating or alleviating infection. The combined use of the agents of the present invention with these other therapies or treatment modalities may be concurrent, or the treatments may be divided up such that the agent of the present invention may be given prior to, after or via a different route than the other therapy or treatment modality.

"Local administration" means direct administration by a non- systemic route at or in the vicinity of the site of a biofilm affliction or infection.

"Therapeutic moieties" refers to any therapeutically effective molecule, whether it is a small organic chemical compound, or a protein or peptide, or a nucleic acid, or an antibody or antibody fragment, or a carbohydrate, that may be attached to the lamellar bodies and administered to subjects suffering from diseases or conditions for which treatment may be beneficial. Optionally, therapeutic moieties can be included on or within the phospholipid bilayers which constitute the lamellar bodies.

### Production of synthetic lamellar bodies

The focus of the present invention is the therapeutic use of lamellar bodies in combination with an antibiotic to treat bacterial infections and/or to inhibit formation of biofilm. The following information provides details of a method for the production of lamellar bodies. The principal phospholipid constituents of lamellar bodies used in the invention are phosphatidylcholine (PC), sphingomyelin (SP), phosphatidylethanolamine (PE), phosphatidylserine (PS), and phosphatidylinositol (PI), and cholesterol.

The lamellar bodies can comprise or consist essentially of these constituents in the range PC 44-70%, SP 15-23%, PE 6-10%, PS 2-6%, PI 2-4%, Cholesterol 4-12%. These figures are percentage by weight.

The preferred composition of phospholipids and cholesterol for phospholipid multilamellar microbodies (lamellar bodies) comprise: PC 55%: SP 19%: PE 8%: PS 4%: PI 3%: cholesterol 10%.

Synthetic lamellar bodies can be prepared by taking a phospholipid mixture, together with cholesterol in the percentages given by weight above, and dissolving these in a chloroform/methanol solvent mixture (2:1 vol/vol). The lipid solution can then be introduced into a round-bottomed flask and attached to a rotary evaporator. The flask is evacuated and rotated at 60 r.p.m. in a thermostatically controlled waterbath at a temperature of 30°C until a dry lipid film is deposited. Nitrogen is introduced into the flask and the residual solvent removed before its connection to a lyophilizer where it is subjected to a high vacuum at room temperature for one hour. After release of the vacuum and following flushing with nitrogen, saline containing solutes (selected antigen) for entrapment is added. The lipid is hydrated within the flask, flushed with nitrogen, attached to the evaporator, and rotated at 60 r.p.m. at room temperature for thirty minutes. The suspension is allowed to stand for two hours at room temperature to complete the swelling process.

In embodiments, the lamellar bodies can be provided in a therapeutically effective amount with a pharmaceutically acceptable carrier. In one embodiment, the lamellar bodies may be presented in unit dosage forms to facilitate accurate dosing. The term "unit dosage forms" refers to physically discrete units suitable as unitary dosages for human subjects and other animals in particular mammals, each unit containing a predetermined quantity of active material calculated to produce the desired therapeutic effect, in association with a suitable pharmaceutical excipient. Typical unit dosage forms include prefilled, premeasured ampules or syringes of the liquid compositions or pills, tablets, capsules or the like in the case of solid compositions. The unit can be, for example, a single use vial, a pre-filled syringe, a single transdermal patch and the like. The unit dosage form can be in unit dose or unit-of-use packages. As is known to those skilled in the art, a unit dose package is a convenient, patient ready unit. "A unit dosage form could be a 5ml suspension of lamellar bodies in which there would be 55mg of phosphatidylcholine, 19mg of sphingomyelin, 8mg phosphatidylethanolamine, 4mg phosphatidylserine, 3mg phosphatidylinosotol and 10mg cholesterol.

In a particular embodiment, the term "pharmaceutically acceptable" means approved by a regulatory agency of the Federal or a state government or listed in the U. S. Pharmacopeia or other generally recognized pharmacopeia for use in animals, and more particularly in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like.

Sterile isotonic aqueous buffer is a preferred carrier when the pharmaceutical composition is administered intravenously. Saline solutions and aqueous dextrose and glycerol solutions can also be employed as liquid carriers, particularly for injectable solutions.

Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E.W. Martin. Such compositions will contain a therapeutically effective amount of the compound, preferably in purified form, together with a suitable amount of carrier so as to provide the form for proper administration to the subject. The formulation should suit the mode of administration.

In a preferred embodiment, the composition is formulated in accordance with routine procedures as a pharmaceutical composition adapted for intravenous administration to a subject. Typically, compositions for intravenous administration are solutions in sterile isotonic aqueous buffer. Where necessary, the composition may also include a solubilizing agent and a local anaesthetic such as lidocaine to ease pain at the site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The amount of the lamellar body composition and/or lamellar body and antimicrobial combination which will be effective in the treatment of the conditions described herein can be determined by standard clinical techniques based on the present description. In addition, in vitro assays may optionally be employed to help identify optimal dosage ranges. The precise dose to be employed in the formulation will also depend on the route of administration, and the seriousness of the disease or disorder, and should be decided according to the judgement of the practitioner and each subject's circumstances. However, suitable dosage ranges for intravenous administration are generally about 20-500 micrograms of active compound per kilogram body weight. Suitable dosage ranges for intranasal administration are generally about 0.01 pg/kg body weight to 1 mg/kg body weight. Effective doses may be extrapolated from dose-response curves derived from in vitro or animal model test systems.

The invention also provides a pharmaceutical pack or kit comprising one or more containers filled with a composition of the invention. Optionally associated with such container (s) can be a notice in the form prescribed by a governmental agency regulating the manufacture, use or sale of pharmaceuticals or biological products, which notice reflects (a) approval by the agency of manufacture, use or sale for human administration, (b) directions for use, or both.

In a specific embodiment, it may be desirable to administer the compositions of the invention locally to the area in need of treatment; this may be achieved, for example, and not by way of limitation, by local infusion during surgery or by spraying the solution containing the lamellar bodies onto the exposed tissue following surgery, by topical application, by injection, by means of a catheter, or by means of an implant, said implant being of a porous, non-porous, or gelatinous material, including membranes, such as silastic membranes, or fibers or co-polymers such as Elvax (see Ruan et al, 1992, Proc Natl Acad Sci USA, 89:10872-10876). In one embodiment, administration can be by direct injection by aerosolised inhalation.

In yet another embodiment, the lamellar bodies can be delivered in a controlled release system. In one embodiment, a pump may be used (see Langer, supra; Sefton (1987) CRC Crit. Ref. Biomed. Eng. 14:201; Buchwald et al. (1980) Surgery 88:507; Saudek et al. (1989) N. Engl. J. Med. 321:574). In another embodiment, polymeric materials can be used (see Medical Applications of Controlled Release, Langer and Wise (eds.), CRC Pres., Boca Raton, Florida (1974); Controlled Drug Bioavailability, Drug Product Design and Performance, Solen and Ball (eds.), Wiley, New York (1984); Ranger and Peppas, J. (1983) Macromol . Sci. Rev. Macromol. Chem. 23:61; see also Levy et al. (1985) Science 228:190; During et al. (1989) Ann. Neurol. 25:351; Howard et al. (1989) J. Neurosurg. 71:105). In yet another embodiment, a controlled release system can be placed in proximity of the therapeutic target, thus requiring only a fraction of the systemic dose (see, e.g., Goodson, in Medical Applications of Controlled Release (1984) supra, vol. 2, pp. 115-138). Other suitable controlled release systems are discussed in the review by Langer (1990) Science 249: 1527 - 1533. The present invention will now be described with reference to the following examples which are provided for the purpose of illustration and are not intended to be construed as being limiting on the present invention.

### Examples

### Example 1 (for reference)

### Action of synthetic lamellar bodies on expression of genes which form bacterial biofilms

It is known many bacteria, e.g. *Pseudomonas, Burkholderia* and *Staphylococcus,* create biofilms that serve to protect the bacterium from the host's immune defence and antimicrobials.

In a mouse model of *Pseudomonas* infection, mice were intranasally infected with LESB65, a strain of *P. aeruginosa,* (2 x 10⁶ CFU in 50µl PBS) and LMS-611 treated mice were given LMS-611 at 1mg per dose.

Bacterial RNA was isolated from mouse lung and nasopharynx at days 0, 1, 2, 3 and 7.

Gene expression profiles for the control (LMS-611 untreated) mice and mice treated with LMS-611 were completed by qPCR (quantitative polymerase chain reaction) analyses which allows a "snapshot" of expressed genes (messenger RNA) within cells.

LMS-611 untreated samples showed up-regulation of genes associated with exopolysaccharide (a biofilm building block) production and quorum sensing. In particular, pelB (exopolysaccharide associated with biofilm formation) flgD (flagellum/motility associated gene), algD (biofilm alginate gene) and lasR (central gene in quorum sensing) are increased in the untreated samples, and significantly reduced in the samples from LMS-611 treated mice.

In Figure 1B it is indicated that in LMS-611 untreated samples, flgD and pelB show a marked increase in expression during the seven day period. Whilst there is an increase in pelB in the treated samples this is reduced in relation to the untreated control sample. The expression of these markers was normalised to proC a reference gene and thus the numbers are unaffected by the increase in the bacterial numbers observed.

This data supports the determination that LMS-611 inhibits biofilm formation through interference with the quorum sensing machinery.

It may be advantageous to supply low levels of LMS-611 e.g. (30-40µg/ml of LMS-611) as for the lung, antibiotics are nebulised and it can take 20 to 30 minutes to do this, thus if the volume is reduced this treatment time, volume of antibiotics and associated side effects can all be reduced. Similarly for the eye, there is only so much volume that the eye can accommodate, thus reductions could be beneficial.

### Example 2 (for reference)

In vitro, Artificial Sputum Models (ASM), considered to represent biofilm typical of that seen in CF, were used to study *Pseudomonas aeruginosa* infection. Pyocyanin, a blue-green phenazine pigment produced by *P. aeruginosa* and controlled by the quorum sensing system (a system of inter-bacterial signalling which correlates to population density) and thus its presence and concentration in a bacterial culture can be used to give an indication of the strength of quorum sensing activity. The levels detected in a PA infected ASM exposed to a range of concentrations of LMS-611 were measured.

As demonstrated in Figure 2, pyocyanin levels detected in the artificial sputum cultures suggests that pyocyanin availability is reduced in LMS-611 treated cultures - the blue green of the pyocyanin can be seen to be progressively less intense in those images taken of wells treated with higher concentrations of LMS-611. Levels of available pyocyanin are reduced from 10mg/ml to 0.3125mg/ml. *Scanning electron microscopy (SEM) images of Pseudomonas* and *Burkholderia* colonies were grown in the presence and absence of LMS-611. Figure 9 illustrates that where LMS-611 was present, bacterial biofilm was eradicated. Figure 11 Scanning electron microscopy (SEM) images (5000 x magnification) of untreated biofilms of PA01 (A) grown for 24h and biofilms of PA01 grown for 24h treated with LMS-611 reconstituted in physiological saline at 40mg/ml (B), 1.25mg/ml (C) and 0.325mg/ml (D & E) for 18h. The untreated control biofilm (A) shows multiple layers of rod-shaped *P. aeruginosa* cells encased within the web-like matrix of the biofilm which consists of polysaccharides, proteins and DNA. In the biofilm treated with the highest dose of LMS-611 (40mg/ml) (B), the LMS appears to coat the surface of the biofilm and there seems to be a reduction in the 3D structure. The biofilms treated with 1.25mg/ml LMS-611 (C) and 0.325mg/ml (D) displayed a clear reduction in the number of cells present in comparison to the multi-layered untreated biofilm (A). In the magnified image of the biofilm treated with 0.325mg/ml (E), not only are the number of *P. aeruginosa* cells reduced but the cells that remain have an altered morphology. Instead of healthy rod-shaped cells many of them have become damaged by treatment.

### Example 3 (for reference)

Measurement of metabolic activity of *Pseudomonas aeruginosa* in the Artificial Sputum Model (ASM) using a Resazurin assay, in which the fluorescence detected is directly proportional to the metabolic activity of the bacteria (Figure 3).

The results illustrated in Figure 3 indicate that the *Pseudomonas aeruginosa* remain in an active growth phase in which they are at their most biologically active and most susceptible to the host's immune response and to antimicrobial agents.

This illustrates that LMS-611 has a pro-planktonic effect.

This pro-planktonic effect is further illustrated by Figure 4 when *Pseudomonas aeruginosa* bacteria were grown into a biofilm for three days and then treated for 24 hours with different concentrations of LMS-611, increased numbers of bacteria were determined for LMS-611-treated biofilms. The artificial sputum model appears to agree with the mouse model. Colony forming units per ml significantly increase following LMS-611 treatment of a formed biofilm compared to the untreated control. The increase appears to be dose dependent with the greatest increase in cultures treated with higher concentrations of LMS-611 (10-20 mg/ml). There was a significant increase in metabolic activity detected by Resazurin in 3 treatments (20 mg/ml, 10 mg/ml and 0.313 mg/ml) demonstrating LMS-611 can cause an increase in the metabolic activity of the cultures.

### Example 4

On a preformed biofilm in an Artificial Sputum Medium, treatment with LMS-611 in combination with ciprofloxacin and tobramycin individually resulted in significantly greater bacterial clearance compared to antibiotic treatment alone (Figure 5).

On a pre-formed biofilm on a MBEC peg plate mature *P. aeruginosa* biofilms were formed for 24h and pre-treated with LMS-611 at a range of concentrations for 1h. Following LMS treatment, biofilms were rinsed thoroughly with physiological saline to remove the LMS. The biofilms were then treated with the antibiotics aztreonam, ciprofloxacin, ceftazidime, meropenem and tobramycin at 1/10 x, 1/5 x and 1 x MIC. The viability (%) of biofilm-associated cells following treatment was assessed using the metabolic XTT assay. LMS pre-treatment appears to increase the susceptibility of cells within the biofilm to the activity of all antibiotics tested.

When LMS-611 was used at 40µg/ml, the antibiotic potentiation appeared most striking, as illustrated in Figure 5 where total bacterial kill was observed for both tobramycin and ciprofloxacin.

This effect was verified *in vivo.* A murine model was developed where mice lungs are infected with *Pseudomonas* and then treated with inhaled LMS-611 or saline. Using Colony Forming Units (CFUs) as the primary variable, it was demonstrated that LMS-611 significantly reduced CFU counts at day 3. Supporting the argument that LMS-611 enhances the body's immune response by preventing concealment of and/or exposing bacteria, it was also demonstrated that a significant increase in macrophage numbers, indicating that the removal of biofilm and or inhibition of its biofilm makes bacteria more visible to immune cells. These are illustrated in Figures 7B and 13.

In the Murine Respiratory Infection Model, groups of BALB/c mice were lightly anesthetised and treated with 50 µl of LMS-611 (20 mg/mL) or vehicle Control (0.9% NaCl) by nasal installation (Carter et al, 2010). Approximately 120 minutes after treatment, all mice were challenged with 1 x 10⁶ colony forming units (CFU) of the bacterium, *Pseudomonas aeruginosa* (LES65B). Treatment with LMS-611 and Control were repeated approximately 24, 48 and 72 hours later. Clinical signs were monitored with the nasopharynx, lungs and blood sampled (5 animals per time point) at five hour post-infection on Day 0 and five hour post-treatment on Days 1, 2, 3 and the infection followed to Day 7 when colony forming units, inflammatory cell infiltrates and pro-inflammatory mediators were measured.

LMS-611, when instilled into the lungs of mice infected with *Pseudomonas aeruginosa* (LES65B), produced a reduction in the number of colony forming units in the nasopharynx and lungs. On Day 3, the number of colony forming units were significantly (p < 0.05) reduced by 1.5 logs (92%) in the LMS-611 treated mice compared to Controls; indicating a much less severe infection. The profile of the Control group infection was consistent with historical data with an infection "spike" on Day 3 (Carter et al, 2010). The infection was followed to Day 7 at which time there was no difference between the treatment groups.

LMS-611 treatment stimulated a significant increase in the number of macrophages compared to the Control group. In contrast, the increases in polymorphonuclear leukocytes, monocyte numbers, macrophage inflammatory protein-2 (MIP2) and tumour necrosis factor (TNF) levels were similar in both groups. The significant reduction in colony forming units in the LMS-611 treated animals appears to be attributable to an increase in the phagocytotic macrophages sufficient to reduce the severity of the bacterial infection and/or to the pro-planktonic effects of LMS-611 increasing the exposure of the bacteria to the inflammatory response of the mice.

This study demonstrates that repeated administration of LMS-611 (20 mg/mL) has an anti-infective effect, significantly reducing the *Pseudomonas aeruginosa* infection induced in the murine lungs.

### Example 5

The present inventors have also investigated whether LMS-611 acts to potentiate the effect of antibiotics. To test this theory, the present inventors determined MIC of various antibiotics, with and without LMS-611 against *Pseudomonas* colonies.

LMS-611 was found to potentiate a range of antibiotics giving scope to improve bacterial clearance and/or use LMS-611/antibiotic combinations at lower doses (or via different routes) with the same efficacy. Given that the use and dose of antibiotics are often restricted by their side effect profile, this also increases the potential use of potent antibiotics whose use would be otherwise limited.

LMS-611 potentiation of antibiotics may be multifactorial. It is considered that in addition to causing bacteria to adopt or remain within a planktonic state, LMS-611 can alter the permeability of bacterial walls potentially rendering them more susceptible to antibiotics and the immune response (see Figure 14).

### Example 6

*Pseudomonas aeruginosa* strains PA01, PA14, two clinical Cystic Fibrosis (CF) strains H183, YH1 and *Burkholderia* cenocepacia strains K56-2, YHBCC5, YHBCC6, YHBCC7 and YHBCC8 were grown on a Nunc Immunosorp PEG plate model for 48 h on a rocking platform. Biofilms were tested in a checkerboard array using LMS-611 (9.8mg/mL) in combination with the antibiotics, piperacillin, aztreonam, meropenem, gentamicin, tobramycin, erythromycin and ciprofloxacin for PA strains and piperacillin, gentamicin, erythromycin and ciprofloxacin for BCC strains. The effect of pre-treating the biofilm with LMS-611 at different time points (5 min, 1, 8 and 24 h) prior to challenging with piperacillin and gentamicin was also investigated. Scanning electron microscopy was used to assess the treated biofilms.

When tested against the PA strains, in combination with a variety of antibiotics used in the treatment of respiratory infections, LMS-611 (9.8mg/kg) was found to significantly reduce the sessile MIC of piperacillin (4-16 fold), gentamicin (4-8 fold) and ciprofloxacin (1-16 fold). Synergy was also demonstrated with aztreonam (2-4 fold), meropenem (1-2 fold) and tobramycin (1-4 fold).

When tested using the BCC strains, in combination with a variety of antibiotics used in the treatment of respiratory infections, LMS-611 (9.8mg/kg) was found to significantly reduce the sessile MIC of piperacillin (4-8 fold), gentamicin (4-8 fold) and ciprofloxacin (2-4 fold). No effect was observed with erythromycin (Table 1 and Table 2).

**Table 1: The sessile minimum inhibitory concentrations (MIC mg/mL) of different antibiotics in the absence and presence of LMS-611 (10mg/mL) against four strains of Pseudomonas aeruginosa using the MBEC assay**

| **PA Strain** | **H183** | | **PA01** | | **PA14** | | **YH1** | |
|---|---|---|---|---|---|---|---|---|
| **Antibiotic** | **MIC** | **MIC +LMS** | **MIC** | **MIC +LMS** | **MIC** | **MIC +LMS** | **MIC** | **MIC +LMS** |
| Piperacillin | 4 | 0.25 | 2 | 0.25 | 8 | 1 | 8 | 1 |
| Meropenem | 2 | 1 | 2 | 2 | 2 | 2 | 2 | 1 |
| Aztreonam | 4 | 2 | 4 | 2 | 4 | 2 | 4 | 1 |
| Gentamicin | 4 | 0.5 | 4 | 0.5 | 4 | 0.5 | 0.5 | 0.125 |
| Tobramycin | <4 | <4 | <4 | <4 | <4 | <4 | 2 | 0.5 |
| Ciporofloxacin | 0.125 | 0.03 | 0.064 | 0.064 | 0.5 | 0.125 | 0.064 | 0.004 |

**Table 2: The sessile minimum inhibitory concentrations (MIC mg/mL) of different antibiotics in the absence and presence of LMS-611 (10mg/mL) against four strains of Burkholderia cenocepacia using the MBEC assay**

| **BCC Strain** | **K56-2** | | **YHBCC5** | | ***YHBCC6*** | | **YHBCC7** | |
|---|---|---|---|---|---|---|---|---|
| **Antibiotic** | **MIC** | **MIC +LMS** | **MIC** | **MIC +LMS** | **MIC** | **MIC +LMS** | **MIC** | **MIC +LMS** |
| Piperacillin | 64 | 16 | 32 | 4 | 32 | 32 | 32 | 4 |
| Gentamicin | 256 | 64 | >256 | 256 | 64 | 16 | 128 | 16 |
| Ciprofloxacin | 8 | 4 | 16 | 4 | 8 | 8 | 8 | 8 |

A comparison of scanning electron micrographs for both the PA and BCC control strains and LMS-611 treated PA and BCC strains revealed that bacterial biofilms were scant and dispersed following treatment with LMS-611.

### Example 7

In a *Pseudomonas* mouse model in which intranasally administered LMS-611 and intravenous tazocin were provided (Drug containing 2 mg piperacillin and 0.5 tazobactam per dose, wherein tazobactam is a beta-lactam inhibitor that prevents resistance to piperacillin), colonies grown from nasopharynx tissue as well as lung tissue isolated 72 hours post infection showed a dramatic decrease in colony counts of *Pseudomonas.* The result for lung tissue is especially surprising for two reasons. Firstly, LMS-611 combined with tazocin gave complete bacterial kill in this *in vivo* model. Secondly, the potentiation of tazocin was achieved even though LMS-611 and tazocin were applied by different routes i.e. intranasally and IV, respectively. This second finding substantiates LMS-611's pro planktonic/ quorum sensing signalling interruption *modus operandi,* which leaves the bacterium open to the effects of the antibiotic and immune defence system (see for example, Figure 7B).

### Example 8

A study was conducted to evaluate the antimicrobial activity of ciprofloxacin and LMS-611 against *Pseudomonas aeruginosa* strain, ATCC 15692 (PA01), in a corneal infection model in C57BL/6 mice. The left cornea was scarified and inoculated with suspensions of *P. aeruginosa* strain ATCC 15692 (PA01) at an inoculum size of 1.79 x 10⁶ CFU/ mouse. Topical treatments of test substances were applied 5 and 10 hrs post infection, 5 µL per application.

The test substances were Ciprofloxacin administered at 0.001% alone (monotherapy) and in combination with LMS-611 (at 10 mg/mL) and LMS-611 administration alone (10 mg/mL).

Animals were euthanized 7.5, 11, 13, 15 and 17 hours post-inoculation and the left eyes were photographed and excised. The bacterial counts were measured as the colony forming units (CFU) per gram of the eye tissue.

The administration of ciprofloxacin monotherapy at 0.001% resulted in a significant reduction in the bacterial counts 7.5, 11, 13, 15 and 17 h post infection compared to the vehicle treatment groups (Figure 15).

The combinations of LMS-611 (10 mg/mL) with ciprofloxacin were also significantly efficacious in reducing bacterial counts 7.5, 11, 13, 15 and 17 h post infection compared to the vehicle control group; however the combinations did not cause significant effect compared to the ciprofloxacin monotherapy groups at all time points.

Treatment with LMS-611 alone at 10 mg/mL was not associated with any significant effect in reducing the bacterial counts compared to the vehicle control group at all of the time points.

### Example 9

A study was conducted to evaluate the antimicrobial activity of ciprofloxacin and LMS-611 against *Pseudomonas aeruginosa* strain, ATCC 15692 (PA01), in a corneal infection model in C57BL/6 mice. The left cornea was scarified and inoculated with suspensions of *P*. *aeruginosa* cells, strain ATCC 15692 (PA01), at an inoculum size of 1.74 x 106/mouse. Topical treatments of test articles were applied 5 and 10 hrs post infection, 5 µL per application. Ciprofloxacin was tested at 0.001% both alone (monotherapy) and in combination with LMS-611 (at 10 mg/mL). LMS-611 when tested alone was dosed at 10 and 20 mg/mL. Animals were euthanized 2, 5, 12, 18, 26 or 36 hours post-inoculation and the left eyes were excised. The bacterial counts were measured as the colony forming units (CFU) per gram of the eye tissue.

The efficacy of ciprofloxacin, 0.001%, in reducing the bacterial counts was time-dependent. Ciprofloxacin monotherapy resulted in a significant reduction in the bacterial counts at 18, 26, and 36 hrs post infection relative to the vehicle treatment groups. However, ciprofloxacin monotherapy did not result in a significant reduction in bacterial counts when measured at 12 hrs post infection. (Figure 16).

The combination treatment of ciprofloxacin with LMS-611 (10 mg/mL) resulted in a significant reduction in the bacterial counts at 12 h post infection. This significant effect was noted relative to the vehicle and the ciprofloxacin alone treatment groups. The combinations of LMS-611 (10 mg/mL) with ciprofloxacin were also significantly efficacious in reducing counts at 18, 26, and 36 h post infection relative to the vehicle control groups; however the combinations did not result in a significant effect relative to the ciprofloxacin monotherapy groups at these later time points.

Treatment with LMS-611 alone, at 10 mg/mL, was not associated with a significant effect in reducing the bacterial counts compared to the vehicle control groups, at all time points tested. The 20 mg/mL dose had a significant, albeit transient, effect on reducing bacterial counts on reducing bacterial counts at the 18 h time point.

## Claims

1. A composition comprising a therapeutically effective amount of lamellar bodies capable of disrupting microbial quorum sensing and microbial biofilm formation and at least a first non-lamellar body antimicrobial active agent, for use in the treatment of a microbial infection wherein the antimicrobial agent is an antibiotic.

2. A composition comprising a therapeutically effective amount of lamellar bodies for use in the treatment of a microbial infection as claimed in claim 1 wherein the microbial infection is of a bacterial infection.

3. A composition comprising a therapeutically effective amount of lamellar bodies for use in the treatment of a microbial infection as claimed in claim 2 for the treatment of a bacterial infection selected from *Pseudomonas, Burkholderia* and *Staphylococcus* spp.

4. A composition comprising a therapeutically effective amount of lamellar bodies for use in the treatment of a microbial infection as claimed in any one of claims 1 to 3 wherein the infection is an infection of the lung or the eye.

5. A composition comprising a therapeutically effective amount of lamellar bodies for use in the treatment of a microbial infection as claimed in any one of claims 1 to 4 wherein the first non-lamellar body antimicrobial active agent is provided to a site of microbial infection by a different route to that of the lamellar bodies, optionally wherein the first non-lamellar body antimicrobial active agent is provided intravenously and the lamellar bodies are provided intranasally.

6. A composition comprising a therapeutically effective amount of lamellar bodies for use in the treatment of a microbial infection as claimed in any one of claims 1 to 5 wherein the at least first antimicrobial active agent and the lamellar bodies are provided in combination separately, sequentially or simultaneously.

7. A kit of parts for use in the treatment of a microbial infection comprising lamellar bodies and a non-lamellar body antibiotic for separate, sequential or simultaneous treatment of microbes to disrupt microbial quorum sensing and microbial biofilm formation.

8. A composition for use in the treatment of a microbial infection as claimed by any one of claims 1 to 6 or a kit as claimed in claim 7 wherein the lamellar bodies comprise phosphatidylcholine, sphingomyelin, phosphatidyl ethanolamine, phosphatidyl serine, phosphatidyl inositol and cholesterol.

9. A composition for use in the treatment of a microbial infection as claimed by any one of claims 1 to 6 or a kit as claimed in claim 7 wherein the lamellar bodies comprise in the range 44-70% phosphatidylcholine, in the range 15-23% sphingomyelin, in the range 6-10% phosphatidyl ethanolamine, in the range 2-6% phosphatidyl serine, in the range 2-4% phosphatidyl inositol and in the range 4-12% cholesterol by weight optionally wherein the lamellar bodies comprise 55% phosphatidylcholine, about 19% sphingomyelin, 8% phosphatidyl ethanolamine, 4% phosphatidyl serine, 3% phosphatidyl inositol and 10% cholesterol by weight.

10. A composition for use in the treatment of a microbial infection as claimed by any one of claims 1 to 6 or a kit as claimed in claim 7 wherein the microbial biofilm is formed by a microbe selected from the group comprising bacteria, viruses, fungi, yeasts and protozoa.

11. A composition for use in the treatment of a microbial infection as claimed by any one of claims 1 to 6 or a kit as claimed in claim 7 wherein the microbial biofilm is formed by bacteria, optionally wherein the bacteria are gram negative bacterium.

12. A composition for use in the treatment of a microbial infection as claimed by any one of claims 1 to 6 or a kit as claimed in claim 7 wherein the microbial infection is an implant-associated and/or a catheter- associated and/or an indwelling medical device-associated infection.

13. A composition for use in the treatment of a microbial infection as claimed by any one of claims 1 to 6 or a kit as claimed in claim 7 wherein an antimicrobial active agent used in combination with a lamellar body is an antibiotic selected from the group comprising piperacillin, aztreonam, meropenem, gentamycin, tobramycin, erythromycin, tazocin and ciprofloxacin, ceftazidime or combinations thereof.

## Patentansprüche

1. Eine Zusammensetzung, beinhaltend eine therapeutisch wirksame Menge von lamellaren Körpern, die in der Lage sind, das mikrobielle Quorum-Sensing und die mikrobielle Biofilmbildung zu stören, und mindestens einen ersten antimikrobiellen Wirkstoff ohne lamellare Körper zur Verwendung bei der Behandlung einer mikrobiellen Infektion, wobei es sich bei dem antimikrobiellen Wirkstoff um ein Antibiotikum handelt.

2. Eine Zusammensetzung, beinhaltend eine therapeutisch wirksame Menge von lamellaren Körpern zur Verwendung bei der Behandlung einer mikrobiellen Infektion gemäß Anspruch 1, wobei es sich bei der mikrobiellen Infektion um eine bakterielle Infektion handelt.

3. Eine Zusammensetzung, beinhaltend eine therapeutisch wirksame Menge von lamellaren Körpern zur Verwendung bei der Behandlung einer mikrobiellen Infektion gemäß Anspruch 2 zur Behandlung einer bakteriellen Infektion, ausgewählt aus *Pseudomonas, Burkholderia* und *Staphylococcus* spp.

4. Eine Zusammensetzung, beinhaltend eine therapeutisch wirksame Menge von lamellaren Körpern zur Verwendung bei der Behandlung einer mikrobiellen Infektion gemäß einem der Ansprüche 1 bis 3, wobei es sich bei der Infektion um eine Infektion der Lunge oder des Auges handelt.

5. Eine Zusammensetzung, beinhaltend eine therapeutisch wirksame Menge von lamellaren Körpern zur Verwendung bei der Behandlung einer mikrobiellen Infektion gemäß einem der Ansprüche 1 bis 4, wobei der erste antimikrobielle Wirkstoff ohne lamellare Körper über einen anderen Weg als bei den lamellaren Körpern an einer Stelle einer mikrobiellen Infektion bereitgestellt wird, wobei optional der erste antimikrobielle Wirkstoff ohne lamellare Körper intravenös bereitgestellt wird und die lamellaren Körper intranasal bereitgestellt werden.

6. Eine Zusammensetzung, beinhaltend eine therapeutisch wirksame Menge von lamellaren Körpern zur Verwendung bei der Behandlung einer mikrobiellen Infektion gemäß einem der Ansprüche 1 bis 5, wobei der mindestens erste antimikrobielle Wirkstoff und die lamellaren Körper in Kombination getrennt, sequenziell oder gleichzeitig bereitgestellt werden.

7. Ein Kit mit Teilen zur Verwendung bei der Behandlung einer mikrobiellen Infektion, beinhaltend lamellare Körper und ein Antibiotikum ohne lamellare Körper zur getrennten, sequentiellen oder gleichzeitigen Behandlung von Mikroben, um das mikrobielle Quorum-Sensing und die mikrobielle Biofilmbildung zu stören.

8. Eine Zusammensetzung zur Verwendung bei der Behandlung einer mikrobiellen Infektion gemäß einem der Ansprüche 1 bis 6 oder ein Kit gemäß Anspruch 7, wobei die lamellaren Körper Phosphatidylcholin, Sphingomyelin, Phosphatidylethanolamin, Phosphatidylserin, Phosphatidylinositol und Cholesterin beinhalten.

9. Eine Zusammensetzung zur Verwendung bei der Behandlung einer mikrobiellen Infektion gemäß einem der Ansprüche 1 bis 6 oder ein Kit gemäß Anspruch 7, wobei die lamellaren Körper Folgendes beinhalten: im Bereich von 44-70 Gew.-% Phosphatidylcholin, im Bereich von 15-23 Gew.-% Sphingomyelin, im Bereich von 6-10 Gew.-% Phosphatidylethanolamin, im Bereich von 2-6 Gew.-% Phosphatidylserin, im Bereich von 2-4 Gew.-% Phosphatidylinositol und im Bereich von 4-12 Gew.-% Cholesterin, wobei die lamellaren Körper optional Folgendes beinhalten: 55 Gew.-% Phosphatidylcholin, 19 Gew.-% Sphingomyelin, 8 Gew.-% Phosphatidylethanolamin, 4 Gew.-% Phosphatidylserin, 3 Gew.-% Phosphatidylinositol und 10 Gew.-% Cholesterin.

10. Eine Zusammensetzung zur Verwendung bei der Behandlung einer mikrobiellen Infektion gemäß einem der Ansprüche 1 bis 6 oder ein Kit gemäß Anspruch 7, wobei der mikrobielle Biofilm durch eine Mikrobe gebildet ist, die aus der Gruppe, bestehend aus Bakterien, Viren, Pilzen, Hefen und Protozoen, ausgewählt ist.

11. Eine Zusammensetzung zur Verwendung bei der Behandlung einer mikrobiellen Infektion gemäß einem der Ansprüche 1 bis 6 oder ein Kit gemäß Anspruch 7, wobei der mikrobielle Biofilm durch Bakterien gebildet ist, wobei es sich bei den Bakterien optional um ein gramnegatives Bakterium handelt.

12. Eine Zusammensetzung zur Verwendung bei der Behandlung einer mikrobiellen Infektion gemäß einem der Ansprüche 1 bis 6 oder ein Kit gemäß Anspruch 7, wobei es sich bei der mikrobiellen Infektion um eine implantatassoziierte und/oder eine katheterassoziierte und/oder eine mit einer medizinischen Verweilvorrichtung assoziierte Infektion handelt.

13. Eine Zusammensetzung zur Verwendung bei der Behandlung einer mikrobiellen Infektion gemäß einem der Ansprüche 1 bis 6 oder ein Kit gemäß Anspruch 7, wobei ein antimikrobieller Wirkstoff, der in Kombination mit einem lamellaren Körper verwendet wird, ein Antibiotikum ist, das aus der Gruppe, beinhaltend Piperacillin, Aztreonam, Meropenem, Gentamycin, Tobramycin, Erythromycin, Tazocin und Ciprofloxacin, Ceftazidim oder Kombinationen davon, ausgewählt ist.

## Revendications

1. Une composition comprenant une quantité thérapeutiquement efficace de corps lamellaires capables de perturber la détection de quorum microbien et la formation de biofilm microbien et au moins un premier agent actif antimicrobien n'étant pas à base de corps lamellaires, pour une utilisation dans le traitement d'une infection microbienne, l'agent antimicrobien étant un antibiotique.

2. Une composition comprenant une quantité thérapeutiquement efficace de corps lamellaires pour une utilisation dans le traitement d'une infection microbienne telle que revendiquée dans la revendication 1, l'infection microbienne étant une infection bactérienne.

3. Une composition comprenant une quantité thérapeutiquement efficace de corps lamellaires pour une utilisation dans le traitement d'une infection microbienne telle que revendiquée dans la revendication 2 pour le traitement d'une infection bactérienne sélectionnée parmi *Pseudomonas, Burkholderia* et *Staphylococcus* spp.

4. Une composition comprenant une quantité thérapeutiquement efficace de corps lamellaires pour une utilisation dans le traitement d'une infection microbienne telle que revendiquée dans n'importe laquelle des revendications 1 à 3, l'infection étant une infection du poumon ou de l'oeil.

5. Une composition comprenant une quantité thérapeutiquement efficace de corps lamellaires pour une utilisation dans le traitement d'une infection microbienne telle que revendiquée dans n'importe laquelle des revendications 1 à 4 où le premier agent actif antimicrobien n'étant pas à base de corps lamellaires est fourni à un site d'infection microbienne par une voie différente de celle des corps lamellaires, facultativement où le premier agent actif antimicrobien n'étant pas à base de corps lamellaires est fourni par voie intraveineuse et les corps lamellaires sont fournis par voie intranasale.

6. Une composition comprenant une quantité thérapeutiquement efficace de corps lamellaires pour une utilisation dans le traitement d'une infection microbienne telle que revendiquée dans n'importe laquelle des revendications 1 à 5 où l'au moins premier agent actif antimicrobien et les corps lamellaires sont fournis en combinaison séparément, séquentiellement ou simultanément.

7. Un kit d'éléments pour une utilisation dans le traitement d'une infection microbienne comprenant des corps lamellaires et un antibiotique n'étant pas à base de corps lamellaires pour un traitement séparé, séquentiel ou simultané de microbes afin de perturber la détection de quorum microbien et la formation de biofilm microbien.

8. Une composition pour une utilisation dans le traitement d'une infection microbienne telle que revendiquée par n'importe laquelle des revendications 1 à 6 ou un kit tel que revendiqué dans la revendication 7 où les corps lamellaires comprennent de la phosphatidylcholine, de la sphingomyéline, de la phosphatidyléthanolamine, de la phosphatidylsérine, du phosphatidylinositol et du cholestérol.

9. Une composition pour une utilisation dans le traitement d'une infection microbienne telle que revendiquée par n'importe laquelle des revendications 1 à 6 ou un kit tel que revendiqué dans la revendication 7 où les corps lamellaires comprennent de la phosphatidylcholine dans la gamme de 44 à 70 %, de la sphingomyéline dans la gamme de 15 à 23 %, de la phosphatidyléthanolamine dans la gamme de 6 à 10 %, de la phosphatidylsérine dans la gamme de 2 à 6 %, du phosphatidylinositol dans la gamme de 2 à 4 % et du cholestérol dans la gamme de 4 à 12 % en poids, facultativement où les corps lamellaires comprennent 55 % de phosphatidylcholine, 19 % de sphingomyéline, 8 % de phosphatidyléthanolamine, 4 % de phosphatidylsérine, 3 % de phosphatidylinositol et 10 % de cholestérol en poids.

10. Une composition pour une utilisation dans le traitement d'une infection microbienne telle que revendiquée par n'importe laquelle des revendications 1 à 6 ou un kit tel que revendiqué dans la revendication 7 où le biofilm microbien est formé par un microbe sélectionné dans le groupe comprenant des bactéries, des virus, des champignons, des levures et des protozoaires.

11. Une composition pour une utilisation dans le traitement d'une infection microbienne telle revendiquée par n'importe laquelle des revendications 1 à 6 ou un kit tel que revendiqué dans la revendication 7 où le biofilm microbien est formé par des bactéries, facultativement où les bactéries sont une bactérie à Gram négatif.

12. Une composition pour une utilisation dans le traitement d'une infection microbienne telle que revendiquée par n'importe laquelle des revendications 1 à 6 ou un kit tel que revendiqué dans la revendication 7 où l'infection microbienne est une infection associée à un implant et/ou associée à un cathéter et/ou associée à un dispositif médical à demeure.

13. Une composition pour une utilisation dans le traitement d'une infection microbienne telle que revendiquée par n'importe laquelle des revendications 1 à 6 ou un kit tel que revendiqué dans la revendication 7 où un agent actif antimicrobien utilisé en combinaison avec un corps lamellaire est un antibiotique sélectionné dans le groupe comprenant la pipéracilline, l'aztréonam, le méropénème, la gentamicine, la tobramycine, l'érythromycine, le tazocin et la ciprofloxacine, la ceftazidime ou des combinaisons de ceux-ci.
